(19) **Europäisches Patentamt European Patent Office Office européen des brevets**

(11) **EP 4 166 127 A1**

(12) **EUROPEAN PATENT APPLICATION**
published in accordance with Art. 153(4) EPC

(43) Date of publication:
**19.04.2023 Bulletin 2023/16**

(21) Application number: **21821603.4**

(22) Date of filing: **28.04.2021**

(51) International Patent Classification (IPC):
**A61K 6/818** (2020.01)    **A61K 6/831** (2020.01)
**A61K 6/887** (2020.01)

(52) Cooperative Patent Classification (CPC):
**A61K 6/818; A61K 6/831; A61K 6/887**

(86) International application number:
**PCT/JP2021/016906**

(87) International publication number:
**WO 2021/251023 (16.12.2021 Gazette 2021/50)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **12.06.2020   JP 2020102511
27.11.2020   JP 2020196722**

(71) Applicant: **Tokuyama Dental Corporation
Tokyo 110-0016 (JP)**

(72) Inventors:
• **MORISAKI, Hiroshi
Tokyo 110-0016 (JP)**
• **AKIZUMI, Hironobu
Tokyo 110-0016 (JP)**

(74) Representative: **HGF
HGF Europe LLP
Neumarkter Straße 18
81673 München (DE)**

(54) **DENTAL CURABLE COMPOSITION**

(57)    Provided is a dental curable composition containing: (A) a porous organic-inorganic composite filler which comprises an organic resin component obtained by polymerizing a polymerizable monomer component (a) including at least 50 mass% of a polymerizable monomer having a hydrogen-bonding functional group, and an inorganic component composed of a plurality of inorganic particles, and in which the total pore volume of pores having a pore diameter in the range of 1-500 nm, as measured by the nitrogen adsorption method, is 0.01-0.30 cm$^3$/g; (B) a polymerizable monomer component including at least 10 mass% of a polymerizable monomer having a hydrogen-bonding functional group; and (C) a polymerization initiator, wherein (C) the polymerization initiator comprises, as photopolymerization initiators, (c1) a photosensitizer compound, (c2) a reducing agent, and (c3) a photoacid generator composed of an aryliodonium salt compound, and the content of the aryliodonium salt compound is 0.1-5 parts by mass with respect to 100 parts by mass of the total mass of (B) the polymerizable monomer component.

EP 4 166 127 A1

**Description**

TECHNICAL FIELD

The present invention relates to a dental curable composition containing an organic-inorganic composite filler.

BACKGROUND ART

A dental curable composition (hereinafter, sometimes abbreviated as "DCC") is generally a paste-like composition containing a polymerizable monomer (matrix monomer), a filler, and a polymerization initiator as main components. The type, shape, particle diameter, content, and the like of the filler to be used affect the handling property of the dental curable composition, and the aesthetics, mechanical strength, and the like of a cured product obtained by curing.

For example, when an inorganic filler having a large particle diameter is added to a dental curable composition, a cured product is formed with high mechanical strength, whereas the cured product has reduced surface smoothness and wear resistance. As a result, it is difficult to obtain a cured product with a glossy finish surface similar to that of a natural tooth. On the other hand, when a fine inorganic filler having an average particle diameter of 1 $\mu$m or less is added to the dental curable composition, the surface smoothness and wear resistance of the cured product can be improved. However, the viscosity of the dental curable composition is significantly increased since the fine inorganic filler has a large specific surface area. In the treatment of teeth, it needs to adjust the consistency of a dental curable composition to a level suitable for use in the oral cavity. When the amount of a fine inorganic filler added is reduced to lower the consistency, it may cause, for example, an increase in the shrinkage, which is associated with the polymerization of a matrix monomer during the curing of the dental curable composition, and a reduction in the mechanical strength of the resulting cured product.

To avoid such a trade-off relationship, the use of an organic-inorganic composite filler has been proposed (see, for example, Patent Documents 1 and 2). The organic-inorganic composite filler is a composite filler containing a fine inorganic filler in an organic resin. By using the organic-inorganic composite filler, it is possible to maintain excellent surface smoothness and wear resistance in the case of using the fine inorganic filler and reduce the polymerization shrinkage ratio. Note that if the amount of the organic-inorganic composite filler added is too large, the handling property of the paste is deteriorated due to the dryness caused in the paste form. However, by using the organic-inorganic composite filler in combination with an inorganic filler (inorganic particles) having an average particle diameter of 0.1-1 $\mu$m, it is possible to prevent the deterioration of the handing property and form a paste-like dental curable composition having an excellent handling property (see, for example, Patent Document 2).

As a method for producing the above organic-inorganic composite filler, a method is generally employed in which a curable composition obtained by kneading a fine inorganic filler and a polymerizable monomer in advance is polymerized to give a cured product, and then the cured product is ground. However, the composition containing the organic-inorganic composite filler produced in this method has a problem in that the strength of the cured product tends to be low since the bonding at the interface between the matrix and the organic-inorganic composite filler is weak.

An organic-inorganic composite filler having an aggregation gap with a pore volume (volume of pores having a pore diameter in the range of 1-500 nm) of 0.01-0.30 cm³/g, as measured by mercury intrusion porosimetry, (hereinafter, also referred to as "porous organic-inorganic composite filler") has been proposed as an organic-inorganic composite filler that solves this problem (see, for example, Patent Document 3). Patent Document 3 describes that a dental curable composition containing such a porous organic-inorganic composite filler, a polymerizable monomer, and a polymerization initiator not only has good paste handling property and a small polymerization shrinkage ratio and gives a cured product with good surface smoothness and wear resistance but also has improved mechanical strength since the porous organic-inorganic composite filler is retained in the cured product with a high fitting force due to an anchor effect caused by curing after the polymerizable monomer penetrates into aggregation gaps by capillary action.

Furthermore, a method for further improving the mechanical strength by the anchor effect of the porous organic-inorganic composite filler has also been proposed (see, for example, Patent Document 4). Patent Document 4 describes "a dental curable composition including: (A) an organic-inorganic composite filler (excluding an inorganic ultrafine particle filler surface-coated with a silane coupling agent) having an aggregation gap with a pore volume of 0.01-0.30 cm³/g, as measured by a nitrogen adsorption method (herein, the term "pore" refers to a hole having a pore diameter in the range of 1-500 nm); (B) a polymerizable monomer component; and (C) a polymerization initiator, in which an organic resin component of (A) the organic-inorganic composite filler is obtained by polymerizing (a) a polymerizable monomer component, (B) the polymerizable monomer component contains at least 10 mass% of a polymerizable monomer having a hydrogen-bonding functional group, and (a) the polymerizable monomer component contains at least 50 mass% of a polymerizable monomer having a hydrogen-bonding functional group" (hereinafter, also referred to as "hydrogen-bonding DCC"). Patent Document 4 describes that addition of a certain amount of the polymerizable monomer having a hydrogen-bonding functional group (hereinafter, also referred to as "hydrogen-bonding monomer") to (a) the polymerizable monomer component and (B) the polymerizable monomer component, in addition to the anchoring effect, results in the improved mechanical strength of the hydrogen-bonding DCC since hydrogen-bonding interaction occurs between the organic resin component of the organic-inorganic composite filler and the cured product of (B) the polymerizable monomer component, which becomes the organic matrix, in the cured product of the dental curable composition.

Patent Document 1: Japanese Unexamined Patent Application, Publication No.2000-80013
Patent Document 2: PCT International Publication No. WO2015/125470
Patent Document 3: PCT International Publication No. WO2011/115007
Patent Document 4: Japanese Patent No.6219146

DISCLOSURE OF THE INVENTION

Problems to be Solved by the Invention

The present inventors have examined the dental curable composition (hydrogen-bonding DCC) disclosed in Patent Document 4 and found that the desired effects, that is, the effects of providing a cured product having considerably high mechanical strength, wear resistance, and aesthetics can be obtained. On the other hand, the present inventors have found that the stickiness in a paste form of the composition may occur, resulting in deterioration of the handling property thereof, such a phenomenon is remarkable, especially after long-term storage, and the ability to retain the occlusal surface morphology formed when the dental curable composition is filled in a molar Class I cavity or the like is slightly low. In other words, it has been revealed that there is room for improvement in handling and shape retention properties of the dental curable composition containing the porous organic-inorganic composite filler, the polymerizable monomer, and the polymerization initiator disclosed in Patent Document 4.

Therefore, it is an object of the present invention to provide a dental curable composition which provides a cured product having high mechanical strength with good handling and shape retention properties in paste form before curing, and maintains a good handling property for a long time.

Means for Solving the Problems

The present inventors have made earnest studies to attain the above object. As a result, they have found that by using a specific amount of an aryliodonium salt, which is a photoacid generator, as one component of a polymerization initiator (C) to be added to a hydrogen-bonding DCC, the handling property of the paste is greatly improved and good handling property immediately after preparation is maintained even after long-term storage, whereby completing the present invention.

A dental curable composition according to the present invention includes:

a porous organic-inorganic composite filler (A)

which contains:

an organic resin component obtained by polymerizing a polymerizable monomer component (a) including at least 50 mass% of a polymerizable monomer having a hydrogen-bonding functional group, and
an inorganic component composed of a plurality of inorganic particles, and

in which a total pore volume of pores having a pore diameter in a range of 1-500 nm, as measured by a nitrogen adsorption method, is 0.01-0.30 cm$^3$/g;

a polymerizable monomer component (B) including at least 10 mass% of a polymerizable monomer having a hydrogen-bonding functional group; and
a polymerization initiator (C).

In the dental curable composition according to the present invention, the polymerization initiator (C) contains, as photopolymerization initiators, a photosensitizer compound (c1), a reducing agent (c2), and a photoacid generator (c3) composed of an aryliodonium salt compound, and the content of the aryliodonium salt compound is 0.1-5 parts by mass with respect to 100 parts by mass of the total mass of the polymerizable monomer component (B).

Preferably, the organic-inorganic composite filler (A) has a resin layer composed of a cured product of the polymerizable monomer component (a) as the organic resin component, and a plurality of inorganic primary particles having an average particle diameter of 10-1000 nm as the inorganic component, and includes a porous organic-inorganic composite aggregated particle having an aggregate structure in which the plurality of inorganic primary particles are bonded to each other via the resin layer so as to form voids.

Further, the reducing agent (c2) preferably contains a tertiary amine compound and more preferably contains an aromatic tertiary amine compound and an aliphatic tertiary amine compound as the tertiary amine compound.

The dental curable composition according to the present invention preferably further contains an inorganic filler (D) composed of a plurality of inorganic particles having an average particle diameter of 10-1000 nm. In this case, the contents of the organic-inorganic composite filler (A) and the inorganic filler (D) are each preferably 100-300 parts by mass with respect to 100 parts by mass of the total mass of the polymerizable monomer (B), and the total content of the organic-inorganic composite filler (A) and the inorganic filler (D) is preferably 250-550 parts by mass with respect to 100 parts by mass of the total mass of the polymerizable monomer (B). The inorganic particles contained in the organic-inorganic composite filler (A) and the inorganic particles constituting the inorganic filler (D) are both preferably spherical or substantially spherical in shape.

Effects of the Invention

Similarly to the dental curable composition described in Patent Document 3 and the hydrogen-bonding DCC described in Patent Document 4, the dental curable composition according to the present invention has the effect of providing a cured product with low polymerization shrinkage and excellent mechanical strength, wear resistance, and aesthetics due to the use of a porous organic-inorganic composite filler having a specific pore volume as the organic-inorganic composite filler.

Similarly to the hydrogen-bonding DCC, the dental curable composition according to the present invention has the effect of giving a cured product with higher mechanical strength due to an addition of a certain amount of the hydrogen-bonding monomer to each of the organic resin component of the organic-inorganic composite filler and the polymerizable monomer component as a raw material of the organic matrix of the cured product.

Furthermore, the dental curable composition according to the present invention has the effect of improving handling and shape retention properties by using a specific photopolymerization initiator as the polymerization initiator (C), more specifically, by using an aryliodonium salt compound as one component thereof. In other words, a decrease in the handling property due to the occurrence of stickiness in the paste form was observed in the hydrogen-bonding DCC, and the occlusal surface morphology formed when the hydrogen-bonding DCC was filled in a molar Class I cavity or the like was not retained in some cases. On the other hand, the dental curable composition according to the present invention may have suppressed dryness in the paste form and can maintain its excellent handling property for a long time without the occurrence of stickiness even after long-term storage. Moreover, the dental curable composition according to the present invention may have a good shape retention property and can increase the surface hardness of the cured product.

PREFERRED MODE FOR CARRYING OUT THE INVENTION

A dental curable composition according to the present embodiment includes:

    a porous organic-inorganic composite filler (A)

        which contains:

            an organic resin component obtained by polymerizing a polymerizable monomer component (a) including at least 50 mass% of a polymerizable monomer having a hydrogen-bonding functional group, and
            an inorganic component composed of a plurality of inorganic particles, and

            in which the total pore volume of pores having a pore diameter in a range of 1-500 nm, as measured by a nitrogen adsorption method, is 0.01-0.30 cm$^3$/g;

    a polymerizable monomer component (B) including at least 10 mass% of a polymerizable monomer having a hydrogen-bonding functional group; and
    a polymerization initiator (C).

In the dental curable composition according to the present invention, the polymerization initiator (C) contains, as photopolymerization initiators, a photosensitizer compound (c1), a reducing agent (c2), and a photoacid generator (c3) composed of an aryliodonium salt compound, and the content of the aryliodonium salt compound is 0.1-5 parts by mass with respect to 100 parts by mass of the total mass of (B) the polymerizable monomer component.

In other words, the dental curable composition according to the present embodiment is characterized in that the dental curable composition (hydrogen-bonding DCC) described in Patent Document 4 contains a specific photopolymerization initiator as a polymerization initiator (C), thereby improving the handling and shape retention properties of the hydrogen-bonding DCC.

The reason why these properties are improved by using the above-mentioned specific photopolymerization initiator is not always clear, but it is presumed as follows based on the fact confirmed by the present inventors. When a polymerizable monomer component (a), which serves as the organic resin component of the organic-inorganic composite filler, and a polymerizable monomer component (B) of the dental curable composition both do not contain a hydrogen-bonding monomer are used, the cured product is inferior to the hydrogen-bonding DCC in terms of mechanical strength but is often superior in terms of handling and shape retention properties. In addition, in the hydrogen-bonding DCC, the higher the content of the hydrogen-bonding monomer, the lower the handling and shape retention properties. From the above, it is inferred that the reason why the handling and shape retention properties are not good is that an affinity between the organic-inorganic composite filler and the matrix monomer becomes high by the hydrogen bond, the fluidity of the paste is increased, and the stickiness and the shape retention property are decreased. The improvement effect was observed when an aryliodonium salt compound was used as a photoacid generator, and no effect was observed when other compounds were used. Therefore, it is considered that the affinity between the organic resin component of the organic-inorganic composite filler (A) and the polymerizable monomer component (B) is lowered in the paste form in which the aryliodonium salt compound is effectively present, the stickiness is suppressed, and the shape retention property is improved by adding the relatively hydrophilic iodonium salt. In addition, it is presumed that since the affinity during the curing by photopolymerization is the same as that of the hydrogen-bonding DCC, there is no adverse effect on the increase in strength. Furthermore, regarding the increase in the surface hardness of the cured product, it is considered that when the dental curable composition in which the photosensitizer compound, the reducing agent, and the aryliodonium salt compound coexist is irradiated with active light, an aryl radical and an acid (derived from a counter anion) are generated from the aryliodonium salt compound, and a polymerization reaction is accelerated.

Note that Patent Document 4 describes that it is suitable to use a photopolymerization initiator as the polymerization initiator (C), and also does aryliodonium salts as one of the compounds that may be added, if necessary, but not directly discloses a photopolymerization initiator containing a combination of a photosensitizer compound (c1), a reducing agent (c2), and a photoacid generator (c3) and using an aryliodonium compound as the photoacid generator (c3). In other words, the polymerization initiator actually used is only a photopolymerization initiator composed of camphorquinone (CQ) as a photosensitizer compound and ethyl N,N-dimethyl-p-benzoate (DMBE) as a reducing agent, and the aryliodonium salt is merely exemplified in parallel with many other compounds to be available. In addition, in Patent Document 4, the stability of paste properties and the shape retention property are not evaluated, and the challenges in these points are also not recognized

Hereinafter, the dental curable composition according to the present embodiment will be described in detail focusing on each component contained in the dental curable composition according to the present embodiment. Note that in the present specification, the notation "x-y" with numerical values x and y means "x or more and y or less" unless otherwise specified. When a unit is added to only the numerical value y in such notation, the unit is also applied to the numerical value x. In the present specification, the term "(meth)acrylic" means both "acrylic" and "methacrylic". Similarly, the term "(meth)acrylate" means both "acrylate" and "methacrylate", and the term "(meth)acryloyl" means both "acryloyl" and "methacryloyl".

<Organic-inorganic Composite Filler (A)>

The organic-inorganic composite filler means a filler in which an inorganic component (usually, a fine inorganic filler) and an organic resin as an organic component are combined. The organic-inorganic composite filler (A) used in the dental curable composition according to the present embodiment corresponds to (A) the organic-inorganic composite filler in the hydrogen-bonding DCC as described above, and is a porous organic-inorganic composite filler which contains an organic resin component obtained by polymerizing a polymerizable monomer component (a) including at least 50 mass% of a polymerizable monomer having a hydrogen-bonding functional group, and an inorganic component, and in which the total pore volume of pores having a pore diameter in the range of 1-500 nm, as measured by the nitrogen adsorption method, is 0.01-0.30 $cm^3$/g.

Similarly to the porous organic-inorganic composite filler described in Patent Document 3 and the porous organic-inorganic composite filler in the hydrogen-bonding DCC, the organic-inorganic composite filler (A) has a pore volume as described above to make the organic-inorganic composite filler not brittle and easy to produce and to obtain the effect of improving the mechanical strength of the cured product by the anchor effect. From the viewpoint of such effects, the pore volume is preferably 0.03-0.20 $cm^3$/g.

Note that in Patent Document 3, the total pore volume of pores having a pore diameter in the range of 1-500 nm is measured by a mercury intrusion method, but in the present embodiment, as in Patent Document 4, the pore volume is measured by a nitrogen adsorption method that does not require the use of mercury, which requires care in handling. Specifically, the pore volume obtained by calculating the pore diameter distribution by the BJH method from the isothermal adsorption curve measured by the BET method using nitrogen adsorption will be employed. This is because it has been confirmed that the total pore volume of the pores having a pore diameter in the range of 1-500 nm can also be measured by the nitrogen adsorption method, and that, from examination by the present inventors, the pores having a pore diameter in the range of 1-500 nm have an extremely sharp pore distribution with a peak in the vicinity of the pore diameter of 50 nm, and that there are substantially no pores having a pore diameter less than 1 nm, which are difficult to measure by the mercury intrusion porosimetry, or pores having a pore diameter greater than 500 nm, which are difficult to measure by the nitrogen adsorption method. In addition, the average pore diameter of the pores forming the aggregation gap is a median pore diameter determined based on a pore volume distribution in holes having a pore diameter in a range of 1-500 nm, as measured by a nitrogen adsorption method.

Preferably, the organic-inorganic composite filler (A) has a resin layer composed of a cured product of the polymerizable monomer component (a) as the organic resin component, and a plurality of inorganic primary particles having an average particle diameter of 10-1000 nm as the inorganic component, and includes a porous organic-inorganic composite aggregated particle having an aggregate structure in which the plurality of inorganic primary particles are bonded to each other via the resin layer so as to form voids.

Such an organic-inorganic composite filler (A) can be obtained by immersing inorganic aggregated particles, in which inorganic primary particles having an average particle size of 10-1000 nm are aggregated, into a solvent in which the polymerizable monomer components (a) and a polymerization initiator are dissolved, removing the solvent, and then polymerizing and curing the resultant. In this case, the inorganic aggregated particles usually have a spherical shape, a substantially spherical shape, a doughnut shape, or a so-called torus shape such as a dimple shape in which a dent is formed on the surface of the particle, and most of the particles have a curved-surface shape in which the main outer surface is constituted by a curved surface. Then, the organic-inorganic composite aggregated particles constituting the finally obtained organic-inorganic composite filler also have a shape corresponding to the shape of the inorganic aggregated particles.

The content of the organic-inorganic composite filler (A) in the dental curable composition according to the present embodiment is preferably 50-600 parts by mass, and more preferably 100-300 parts by mass with respect to 100 parts by mass of the total mass of the polymerizable monomer component (B) described later, in consideration of difficulty in imparting sufficient strength to the resulting dental curable composition when the content is too small, and the possibility of a deteriorated handling property due to dryness in a paste form when the content is too large.

Hereinafter, the organic resin component and the inorganic filler constituting the organic-inorganic composite filler (A), and the method for producing the organic-inorganic composite filler (A) will be described in detail.

[Organic Resin Component and Polymerizable Monomer Component (a) as Raw Material of the Resin]

In the dental curable composition according to the present embodiment, when the organic-inorganic composite filler (A) is mixed with other components to prepare the dental curable composition in the same manner as the organic-inorganic composite filler of the hydrogen-bonding DCC, a hydrogen bond is formed between the organic-inorganic composite filler (A) and the hydrogen-bonding monomer contained in the polymerizable monomer component (B) described later, and the cured product of the dental curable composition will have further improved mechanical strength by the hydrogen bond. Therefore, the organic resin component in the organic-inorganic composite filler (A) needs to be obtained by polymerizing a polymerizable monomer component (a) including at least 50 mass% of the hydrogen-bonding monomer (mass% is based on the total mass of the polymerizable monomer component (a)).

When the content ratio of the hydrogen-bonding monomer is less than 50%, it is difficult to obtain the effect of improving the mechanical strength. From the viewpoint of the effect of improving mechanical strength, the proportion of the hydrogen bonding monomer in the polymerizable monomer component (a) is preferably 70-100 mass%. Note that when the amount of the hydrogen-bonding monomer in the polymerizable monomer component (a) is less than 100 mass%, the polymerizable monomer component (a) contains a polymerizable monomer other than the hydrogen-bonding monomer (hereinafter, also referred to as "non-hydrogen-bonding monomer"), and the amount thereof is the balance of the amount of the hydrogen-bonding monomer. Hereinafter, the hydrogen-bonding monomer and the non-hydrogen-bonding monomer will be described.

(Hydrogen-bonding Monomer)

The "hydrogen-bonding functional group" of the hydrogen-bonding monomer means a functional group that causes a hydrogen bond, that is, a bonding interaction formed between a hydrogen atom (acceptor) polarized to be electrically positive by bonding to an atom (e.g., O, N, and S) having high electronegativity and an electrically negative atom (donor) having a lone electron pair. The substituent having a portion functioning as a donor and a portion functioning as an acceptor in the hydrogen bond is a hydrogen-bonding functional group, and specifically, a hydroxy group (-OH), an amino group (-NH$_2$), a thiol group (-SH), a urethane group {urethane bond: - NHC(=O)O-}, an amide group {carbamoyl group: -C(=O)NH$_2$} and the like.

The hydrogen-bonding monomer is not particularly limited as long as it is a compound having the above hydrogen-bonding functional group and a polymerizable group. For example, a cationic polymerizable monomer such as an epoxy compound or an oxetane compound; and a radical polymerizable monomer can be used. From the viewpoint of reducing the polymerization shrinkage ratio, a cationic polymerizable monomer capable of ring-opening polymerization is suitably used, while from the viewpoint of low biotoxicity and high polymerization activity, a radical polymerizable monomer is suitably used.

Examples of the radical polymerizable hydrogen-bonding monomer that can be suitably used include monofunctional vinyl monomers such as methacrylic acid, acrylic acid, p-methacryloyloxybenzoic acid, N-2-hydroxy-3-methacryloyloxypropyl-N-phenylglycine, 4-methacryloyloxyethyl trimellitic acid, 6-methacryloyloxyhexamethylenemalonic acid, 10-methacryloyloxydecamethylenemalonic acid, 2-methacryloyloxyethyl dihydrogen phosphate, and 10-methacryloyloxydecamethylene dihydrogen phosphate; and bifunctional vinyl monomers such as 2,2-bis[4-(3-methacryloyloxy)-2-hydroxypropoxyphenyl]propane and the corresponding acrylate, 1,2-bis(3-methacryloyloxy-2-hydroxypropoxy)ethyl, and 1,6-bis(methacrylethyloxycarbonylamino)trimethylhexane.

Among them, it is preferable to use a radical polymerizable monomer having two or more hydrogen-bonding functional groups in one molecule, specifically, 2,2-bis[4-(3-methacryloyloxy)-2-hydroxypropoxyphenyl]propane (GMA), 1,6-bis(methacrylethyloxycarbonylamino)trimethylhexane (UDMA) or the like. These hydrogen-bonding monomers may be used alone or in a combination of two or more thereof.

(Non-hydrogen-bonding Monomer)

As the non-hydrogen-bonding monomer, a polymerizable monomer used in a dental curable composition can be used without particular limitation as long as it is a compound having a polymerizable group, that is, a compound being cationically polymerizable or radically polymerizable, and not having a hydrogen-bonding functional group. A (meth)acrylic polymerizable monomer is preferable because the resulting polymer has good mechanical strength, biological safety, and the like. In addition, it is preferable to use a polymerizable monomer having a plurality of polymerizable functional groups in the molecule (bifunctional or more), especially bi- to tetra-functional polymerizable monomer for reasons such as higher degree of polymerization and particularly high mechanical physical properties of the cured product.

Examples of the non-hydrogen-bonding monomer that can be suitably used include ethylene glycol dimethacrylate, diethylene glycol dimethacrylate, triethylene glycol dimethacrylate, butylene glycol dimethacrylate, neopentyl glycol dimethacrylate, 1,3-butanediol dimethacrylate, 1,4-butanediol dimethacrylate, 1,6-hexanediol dimethacrylate, 2,2-bis(methacryloyloxyphenyl)propane, 2,2-bis[(3-methacryloyloxy-2-hydroxypropyloxy)phenyl]propane, 2,2-bis(4-methacryloyloxyphenyl)propane, 2,2-bis(4-methacryloyloxypolyethoxyphenyl)propane, 2,2-bis(4-methacryloyloxydiethoxyphenyl)propane, trimethylolpropane trimethacrylate, trimethylolethane trimethacrylate, pentaerythritol trimethacrylate, and pentaerythritol tetramethacrylate. These non-hydrogen-bonding monomers may be used alone or in a combination of two or more thereof.

[Inorganic Filler]

As the inorganic filler, it is preferable to use an inorganic filler containing a plurality of inorganic primary particles having an average particle diameter of 10-1000 nm, particularly an aggregated filler in which such inorganic primary particles are aggregated. As the inorganic primary particles, particles composed of inorganic oxides such as amorphous silica, silica-zirconia, silica-titania, silica-titania-barium oxide, silica-titania-zirconia, quartz, alumina, titania, zirconia, and glass can be used. Among them, silica-based composite oxide particles, particularly silica-zirconia particles are preferable. Spherical or substantially spherical particles having an average uniformity of 0.6 or more are preferably used because the organic-inorganic composite filler has excellent wear resistance and surface smoothness, uniform pores, and an opening thereof that is closed by the organic resin phase so that air bubbles are hardly enclosed therein. The inorganic primary particles may have an average particle diameter of 10-1000 nm and preferably 40-800 nm and more preferably 50-600 nm.

[Method for Producing Organic-inorganic Composite Filler(A)]

The organic-inorganic composite filler (A) can be suitably produced by immersing inorganic aggregated particles obtained by subjecting the above-described inorganic primary particles or aggregated particles thereof to surface treatment with a silane coupling agent if necessary and then spray-drying in a polymerizable monomer solution prepared by dissolving the polymerizable monomer component (a) and a polymerization initiator in an organic solvent, removing the organic solvent, and then polymerizing and curing the resultant.

Examples of the silane coupling agent used in the surface treatment performed, if necessary, include methyltrimethoxysilane, methyltriethoxysilane, methyltrichlorosilane, dimethyldichlorosilane, trimethylchlorosilane, vinyltrichlorosilane, vinyltriethoxysilane, vinyltris(β-methoxyethoxy) silane, γ-methacryloyloxypropyltrimethoxysilane, γ-chloropropyltrimethoxysilane, γ-glycidoxypropyltrimethoxysilane, and hexamethyldisilazane. In addition, the spray drying in the surface treatment refers to a method in which inorganic particles are dispersed in a volatile liquid medium such as water to form a slurry, the slurry is finely atomized by a high-speed gas stream or the like, this atomized material is brought into contact with a high-temperature gas to volatilize the liquid medium, and the inorganic particles dispersed in liquid droplets are substantially collected into one aggregated particle to form inorganic aggregated particles. According to this method, the particle diameter and the particle size distribution of the aggregated particles can be flexibly controlled by the spraying mode and the spraying conditions.

As the organic solvent used in the polymerizable monomer solution in which the inorganic aggregated particles are immersed, methanol, ethanol, acetone, dichloromethane, or the like can be suitably used. The content of the polymerizable monomer component (a) in the polymerizable monomer solution is preferably 10-50 parts by mass with respect to 100 parts by mass of the organic solvent. In addition, as the polymerization initiator, it is preferable to use a thermal polymerization initiator from the viewpoint of not selecting a working environment such as under light shielding or red light, and an azo compound such as azobisisobutyronitrile with high safety in handling and little influence on the coloration of the organic-inorganic composite filler is suitably used. The amount of the polymerization initiator added is usually 0.1-5 parts by mass with respect to 100 parts by mass of the polymerizable monomer component (a).

In the immersion of the inorganic aggregated particles into the polymerizable monomer solution, the inorganic aggregated particles may be mixed in the polymerizable monomer solution such that the amount of the polymerizable monomer is 30-500 parts by mass, particularly 50-200 parts by mass with respect to 100 parts by mass of the inorganic aggregated particles. After that, the mixture is preferably left for 1 hour or more.

Removal of the organic solvent is preferably carried out until substantially all of the organic solvent (usually 95 mass% or more) is removed and a visually free-flowing powder is obtained. The removal of the organic solvent is not particularly limited as long as it is a method capable of such removal and can be suitably performed by reduced pressure drying (or vacuum drying) in which drying is performed under a reduced pressure of, for example, 0.01-50 hPa, particularly 0.1-10 hPa. The polymerization and curing may be carried out by appropriately selecting a suitable method according to the type of polymerization initiators to be used.

<Polymerizable Monomer Component (B)>

Similarly to the polymerizable monomer component of hydrogen-bonding DCC, the polymerizable monomer component (B) forms hydrogen bonds with the hydrogen-bonding groups contained in the organic resin component of the organic-inorganic composite filler (A) when a dental curable composition is prepared and needs to contain 10 mass% of the hydrogen-bonding monomer (mass% is based on the total mass of the polymerizable monomer component (B)) to further improve mechanical strength in a cured product of the dental curable composition by the hydrogen bonds. When the content of the hydrogen-bonding monomer is increased, the viscosity of the dental curable composition according to the present embodiment tends to increase. From the viewpoint of making the viscosity easy to handle during tooth treatment, the content of the hydrogen-bonding monomer in the polymerizable monomer component (B) is preferably 10-70 mass%. In this case, the polymerizable monomer other than the hydrogen-bonding monomer will be occupied by non-hydrogen-bonding monomers.

The same hydrogen-bonding and non-hydrogen-bonding monomers can be used as the hydrogen-bonding and non-hydrogen-bonding monomers in the polymerizable monomer component (a). The same is also applicable in that (meth) acrylic polymerizable monomers are preferable in all of the cured products obtained because of, for example, their good mechanical strength and biological safety and that bi- or more functional polymerizable monomers, especially bi- to tetra-functional polymerizable monomers are preferable for the reasons of the higher degree of polymerization and the increased mechanical physical properties of the cured product.

The content of the polymerizable monomer component (B) in the dental curable composition according to the present embodiment is preferably 10-35 mass% and more preferably 15-30 mass%.

<Polymerization Initiator (C)

In the dental curable composition according to the present embodiment, a photopolymerization initiator including a photosensitizer compound (c1), a reducing agent (c2), and a photoacid generator (c3) composed of an aryliodonium salt compound is used as the polymerization initiator (C), and the amount of the aryliodonium salt compound added needs to be 0.1-5 parts by mass with respect to 100 parts by mass of the total mass of the polymerizable monomer component (B).

The aryliodonium salt compound functions not only as a photoacid generator but also as a modifier that improves the handling and shape retention properties of the paste. It is presumed that the latter function is achieved by (temporarily) reducing the affinity between the organic resin component of the organic-inorganic composite filler (A) and the polymerizable monomer component (B). When a compound other than the aryliodonium salt compound is used as the photoacid generator, it is difficult to obtain such an effect. Hereinafter, each component constituting the photopolymerization initiator will be described.

[Photosensitizer Compound (c1)]

As the photosensitizer compound (c1), a compound having a maximum absorption wavelength of 350-700 nm and having a function of generating an active species effective for polymerization can be used without particular limitation. Here, the active species means a neutral radical species with high activity, usually resulting from energy transfer or electron transfer between a photosensitizer compound excited through light absorption and a polymerizable monomer or another compound.

Examples of compounds particularly preferably used as the photosensitizer compound include benzoin alkyl ethers such as benzoin methyl ether, benzoin ethyl ether, and benzoin isopropyl ether; benzyl ketals such as benzyl dimethyl ketal and benzyl diethyl ketal; benzophenones such as benzophenone, 4,4'-dimethylbenzophenone, and 4-methacryloxybenzophenone; $\alpha$-diketones such as camphorquinone, 9,10-phenanthraquinone, benzyl, diacetyl, acetylbenzoyl, 2,3-pentadione, 2,3-octadione, 4,4'-dimethoxybenzyl, acenaphthenequinone, 2,3-pentadionebenzyl, and 9,10-anthraquinone; thioxanthones such as 2,4-diethylthioxanthone, 2-chlorothioxanthone, and methylthioxanthone; and bisacylphosphine oxides such as bis-(2,6-dichlorobenzoyl)phenylphosphine oxide, bis(2,6-dichlorobenzoyl)-2,5-dimethylphenylphosphine oxide, bis-(2,6-dichlorobenzoyl)-4-propylphenylphosphine oxide, bis-(2,6-dichlorobenzoyl)-1-naphthylphosphine oxide, and bis(2,4,6-trimethylbenzoyl)-phenylphosphine oxide. Among them, $\alpha$-diketones are preferably used. These photosensitizer compounds may be used alone or in a combination of two or more thereof.

The content of the photosensitizer compound is usually 0.01-10 parts by mass, preferably 0.03-5 parts by mass, and more preferably 0.05-2 parts by mass with respect to 100 parts by mass of the total mass of the polymerizable monomer component (B).

[Reducing Agent (c2)]

The reducing agent (or electron donor) exhibits a polymerization accelerating function when used in combination with the photosensitizer compound. Although any known reducing agent can be used without limitation, it is preferable to use a tertiary amine compound because its high polymerization potential can be obtained. As the tertiary amine compound, an aliphatic tertiary amine compound having no aromatic ring in the molecule or an aromatic tertiary amine compound having an aromatic ring in the molecule can be used, and it is preferable to use the aromatic tertiary amine compound. It is particularly preferable to use an aromatic tertiary amine compound and an aliphatic tertiary amine compound in combination because high polymerization activity is exhibited, polymerization curability by irradiation light in a short time is maintained, high physical properties of a cured product are exhibited, and coloration, when the cured product is exposed to ultraviolet light such as sunlight, can be reduced.

Examples of the aromatic tertiary amine compound that can be suitably used include N,N-dimethylaniline, N,N-dibenzylaniline, N,N-dimethyl-p-toluidine, N,N-diethyl-p-toluidine, methyl p-(N,N-dimethyl)aminobenzoate, ethyl p-(N,N-dimethyl)aminobenzoate, and amyl p-(N,N-dimethyl)aminobenzoate. Examples of the aliphatic tertiary amine compound that can be suitably used include triethanolamine, N-methyldiethanolamine, triethylamine, tributylamine, N,N-dimethylaminoethyl methacrylate, and N,N-diethylaminoethyl methacrylate. These aromatic tertiary amine compounds and/or aliphatic tertiary amine compounds may each be used alone or in a combination of two or more thereof.

The content of the reducing agent (c2) is usually 0.01-10 parts by mass, preferably 0.03-5 parts by mass, and more preferably 0.05-2 parts by mass with respect to 100 parts by mass of the total mass of the polymerizable monomer component (B) .

[Photoacid Generator (c3): Aryliodonium Salt Compound]

As the aryliodonium salt compound to be used as the photoacid generator (c3), any known aryliodonium salt compound that functions as a photoacid generator can be used without particular limitation. Examples of the aryliodonium salt that can be suitably used include salts composed of a cation such as diphenyliodonium, bis(p-chlorophenyl)iodonium, ditolyliodonium, bis(p-methoxyphenyl)iodonium, bis(p-tert-butylphenyl)iodonium, p-isopropylphenyl-p-methylphenyliodonium, bis(m-nitrophenyl)iodonium, p-tert-butylphenylphenyliodonium, p-methoxyphenylphenyliodonium, p-octyloxyphenylphenyliodonium, or p-phenoxyphenylphenyliodonium and an anion such as nitrate, acetate, chloroacetate, carboxylate, or phenolate.

The content of the aryliodonium salt compound needs to be 0.1-5 parts by mass with respect to 100 parts by mass of the polymerizable monomer component (B) in order to impart high curability to a cured product of the dental curable composition and to improve the handling property of the dental curable composition according to the present embodiment in a paste form. When the content of the aryliodonium salt compound (content with respect to 100 parts by mass of the polymerizable monomer component (B)) is less than 0.1 part by mass, it is difficult to obtain the desired effect. In addition, when the above content exceeds 5 parts by mass, not only is excessive use of the aryliodonium salt compound but the effect of improving the mechanical strength of the cured product may not be obtained. From the viewpoint of the effect, the content of the aryliodonium salt compound is preferably 0.2-5 parts by mass and more preferably 0.3-2 parts by mass.

<Inorganic Filler (D)>

The dental curable composition according to the present embodiment preferably contains an inorganic filler (D) because the content of the filler in the dental curable composition is increased and high strength is obtained by containing the inorganic filler in combination with the organic-inorganic composite filler. As the inorganic filler (D), an inorganic filler known as the filling material of a dental restorative material can be used without particular limitation. Typical examples of the material of the inorganic filler include metal oxides such as quartz, silica, alumina, silica-titania, silica-zirconia, lanthanum glass, barium glass, and strontium glass. If necessary, a cation-eluting inorganic filling material known as a dental inorganic filling material, such as silicate glass or fluoroaluminosilicate glass, may be added. These inorganic fillers may be used alone or in a combination of two or more thereof.

The particle diameter of the inorganic filler is not particularly limited, and inorganic fillers having an average particle diameter of 0.01-100 pm, particularly 10-1000 nm, which are generally used as dental materials, can be suitably used. The refractive index of the inorganic filler is also not particularly limited, and a refractive index in a range of 1.4-1.7, which is included in a general dental inorganic filler, can be used without particular limitation and may be set appropriately for the purpose. A plurality of inorganic fillers having different particle diameter ranges, average particle diameters, refractive indices, and materials may be used in combination. The shape of the inorganic filler is not particularly limited, but it is preferable to use a spherical or substantially spherical inorganic filler having an average uniformity of 0.6 or more from the viewpoint of increasing the surface smoothness of the resulting cured product.

The inorganic filler is preferably treated with a surface treatment agent typified by a silane coupling agent for the reasons of better compatibility with polymerizable monomers and improved mechanical strength and water resistance. The surface treatment can be carried out by the same method as described in the method for producing the organic-inorganic composite filler (A).

The content of the inorganic filler (D) may be appropriately determined depending on the content of the organic-inorganic composite filler (A) in consideration of the viscosity when mixed with the polymerizable monomer component and the mechanical strength of the cured product. However, if the content of the inorganic filler (D) is too large, the handling property of the dental curable composition is lowered. Thus, it is preferable that the total content of the organic-inorganic composite filler (A) and the inorganic filler (D) does not exceed 600 parts by mass with respect to 100 parts by mass of the total mass of the polymerizable monomer component (B). In particular, it is preferable that the contents of the organic-inorganic composite filler (A) and the inorganic filler (D) are each 100-300 parts by mass with respect to 100 parts by mass of the total mass of the polymerizable monomer (B), and the total content of the organic-inorganic composite filler (A) and the inorganic filler (D) is 250-550 parts by mass with respect to 100 parts by mass of the total mass of the polymerizable monomer (B).

<Other Additives>

The dental curable composition according to the present embodiment may contain a known additive to the extent that the effect thereof is not impaired. Examples of such additives include a polymerization inhibitor, a pigment, and an ultraviolet absorber.

<Preparation Method and Use of Dental Curable Composition>

The dental curable composition according to the present embodiment can be generally obtained by sufficiently kneading predetermined amounts of each essential component and each optional component to be added if necessary so that the organic-inorganic composite filler (A) and the inorganic filler (D) to be added if necessary are uniformly dispersed in the polymerizable monomer component (B), and degassing the paste under reduced pressure to remove bubbles.

The dental curable composition according to the present embodiment is suitably used as a dental filling and restorative material typified by a photocurable composite resin, particularly as a dental filling and restorative material used for the treatment of a molar to which high occlusal pressure is applied. The dental curable composition can also be used as, for example, dental cement and a restorative material for core construction.

EXAMPLES

Hereinafter, the present invention will be described in more detail with reference to Examples, but the present invention is not limited to these Examples.

1. Abbreviations and Shortened Codes

Listed below are abbreviations for compounds used in Examples and Comparative Examples.

(1) Polymerizable Monomer

<Hydrogen-bonding Monomer>

- GMA: 2,2-bis[(3-methacryloyloxy-2-hydroxypropyloxy)phenyl]propane
- UDMA: 1,6-bis(methacrylethyloxycarbonylamino)trimethylhexane

<Non-hydrogen-bonding Monomer>

·3G: triethylene glycol dimethacrylate
·HD: 1,6-hexanediol dimethacrylate
·D2.6E: 2,2-bis(4-(methacryloyloxyethoxy)phenyl)propane

(2) Inorganic Filler

·F-1: spherical (averageuniformity: 0.95) primary particles of silica-zirconia having an average particle diameter of 200 nm produced by the sol-gel method

(3) Polymerization Initiator

<Thermal Polymerization Initiator>

·AIBN: azobisisobutyronitrile

<Photopolymerization Initiator>

[Photosensitizer Compound]

·CQ: camphorquinone

[Reducing Agent (Tertiary Amine Compound)]

- DMBE: ethyl N,N-dimethyl-p-benzoate
- MDEOA: N-methyldiethanolamine

[Photoacid Generator (Aryliodonium Salt Compound)]

- DPIB: 4-isopropyl-4'-methyldiphenyliodonium tetrakis(pentafluorophenyl) borate
- DPICH: diphenyliodonium-2-carboxylate monohydrate
- DPIFP: bis (4-tert-butylphenyl)iodonium hexafluorophosphate

[Photoacid Generator (Other Than Aryliodonium Salt Compound)]

·TCT: 2,4,6-tris(trichloromethyl)-s-triazine

2. Preparation of Organic-inorganic Composite Filler

Organic-inorganic composite fillers were prepared and evaluated according to the following Production Examples 1-5. The inorganic fillers used and the obtained organic-inorganic composite fillers were evaluated by the following methods.

(1) Method of Measuring Average Particle Diameter and Average Uniformity of Inorganic Filler

A photograph of a powder was taken at a magnification of 5,000-100,000 times by a scanning electron microscope (manufactured by Philips, "XL-30S"), and the image taken was processed using image analysis software (trade name: "IP-1000PC", manufactured by Asahi Kasei Engineering Corporation). The number of particles (30 or more) observed in a unit field of view of the photograph and the primary particle diameter (the maximum diameter) were measured to calculate a number average particle diameter X by the following formula based on the measurement values.

$$\text{Average particle size : } X = \sqrt[3]{\frac{\sum_{i=1}^{n} X_i^3}{n}} \quad \text{(Average volume diameter)}$$

**n : Number of particles observed**
**Xi: Particle size (diameter) of i-th particle**

The number (n: 30 or more) of the particles observed in the unit field of view, the long diameter (Li), which is the maximum diameter of the particles, and the short diameter (Bi), which is the diameter in the direction perpendicular to the long diameter, were also determined to calculate the average uniformity of the inorganic filler with the following formula.

$$\text{Average uniformity} = \frac{\sum_{i=1}^{n} Bi / Li}{n}$$

(2) Method of Measuring Average Particle Diameter (Particle Size) of Organic-inorganic Composite Filler

In 10 ml of ethanol were dispersed 0.1 g of organic-inorganic composite filler and irradiated with ultrasound for 20 minutes. The dispersion was analyzed by laser diffraction/scattering method using a particle size distribution meter ("LS230", manufactured by Beckman Coulter, Inc.) to determine the median diameter through the calculation of volume statistics using an optical model (Fraunhofer).

(3) Method of Measuring Pore Volume of Aggregation Gap in Organic-inorganic Composite Filler

In a sample cell, 0.1 g of the organic-inorganic composite filler was placed and subjected to pretreatment by evacuation at 120°C for 3 hours using a pretreatment apparatus ("Vacu-prep 061", manufactured by Shimadzu Corporation). Thereafter, the total pore volume of pores having a pore diameter in the range of 1-500 nm was determined with a micropore distribution-measuring apparatus based on a gas adsorption method ("TriStar II 3020", manufactured by Shimadzu Corporation) using nitrogen as an adsorption gas and liquid nitrogen as a coolant.

<Production Example 1-4>

The inorganic filler was added to water, and a dispersion having the inorganic filler dispersed therein was obtained by using a circulating bead mill SC Mill. Subsequently, γ-methacryloyloxypropyltrimethoxysilane and acetic acid were added to water and stirred to give a uniform solution with a pH of 4. This solution was added to the inorganic particle dispersion and mixed uniformly. Thereafter, the dispersion was dried by a spray drying method to give an inorganic powder. The spray drying was performed using a spray dryer (Spray Drier "NL-5", manufactured by OHKAWARA KAKOHKI CO.,LTD) in which fine particles were formed by collision with atomizing air at the tip of a nozzle, at a spray pressure of 0.08 MPa and a drying temperature of 230°C. After that, the spray-dried inorganic powder was vacuum-dried at 120°C for 15 hours to give inorganic aggregated particles.

Next, a polymerizable monomer solution was prepared by mixing AIBN as a polymerization initiator and methanol as an organic solvent with a polymerizable monomer mixed at a predetermined quantitative ratio. The inorganic aggregated particles and the polymerizable monomer solution were further mixed so that the polymerizable monomer and the inorganic aggregated particles were at a predetermined ratio, and the mixture was allowed to stand for 1 hour after it was confirmed that the mixture became a slurry state.

The mixture was dried for 1 hour under the conditions of a reduced pressure of 10 hPa and a heating condition of 40°C (using a hot water bath) while being stirred by a rotary evaporator to remove the organic solvent. The removal of the organic solvent gave a free-flowing powder.

The powder was heated for 1 hour under the conditions of a reduced pressure of 10 hPa and a heating condition of 100°C (using an oil bath) while being stirred by a rotary evaporator to polymerize and cure the polymerizable monomer in the powder, thereby giving a porous organic-inorganic composite filler with a curved surface shape. Then, the average particle diameter and the pore volume of the obtained organic-inorganic composite filler were measured. The results are shown in Table 1.

<Production Example 5>

Into a mortar, 100 g of the same inorganic aggregated particles as in Example 1, 12.5 g of GMA as a polymerizable monomer, and 12.5 g of HD as a polymerizable monomer, and AIBN as a polymerization initiator were each put and mixed together to prepare a paste-like mixture. This paste-like mixture was degassed under reduced pressure and then polymerized and cured at 100 °C for 30 minutes. The cured product was ground in a vibratory ball mill (zirconia ball diameter: 5 mm) to give an irregular-shaped nonporous organic-inorganic composite filler. Then, the average particle diameter and the pore volume of the obtained organic-inorganic composite filler were measured. The results are shown in Table 1.

[Table 1]

| Production Example No. | Raw material | | organic-inorganic composite filler | | | | |
| | Inorganic filler: F-1 (parts by mass) | Polymerizable monomer component a (parts by mass) | Classification | Shortened code | Average particle diameter ($\mu$m) | Total pore volume ($cm^3$/g) | Shape |
|---|---|---|---|---|---|---|---|
| 1 | 100 | GMA (20) | Porous | CF-1 | 20 | 0.11 | Curved-surface shape |
| 2 | 100 | GMA (10) /HD (10) | | CF-2 | 20 | 0.10 | Curved-surface shape |
| 3 | 100 | UDMA (20) | | CF-3 | 20 | 0.11 | Curved-surface shape |
| 4 | 100 | UDMA (10) /HD (10) | | CF-4 | 20 | 0.10 | Curved-surface shape |
| 5 | 100 | GMA (12.5) /HD (12.5) | Nonporous | CF-5 | 30 | 0.00 | Irregular-shape |

3. Preparation and Evaluation of Dental Curable Composition

Dental curable compositions were prepared and evaluated according to Examples 1-14 and Comparative Examples 1-6 described below. The dental curable compositions were evaluated by the following methods.

(1) Method for Evaluating Handling Property of Paste

The paste properties of the dental curable compositions before curing were evaluated based on the following criteria from the viewpoint of handling property. In other words, samples with little stickiness were rated as "B", with particularly little stickiness as "A", and with strong stickiness and difficulty in handling as "C". Furthermore, samples with little dryness were rated as "B", with particularly little dryness as "A", and with strong dryness and difficulty in handling as "C". Evaluations were performed immediately after the dental curable compositions were prepared and after storage at 37°C for 6 months.

(2) Method for Evaluating Shape Retention Property of Paste

The paste shape-retention properties of the dental curable compositions before curing were evaluated by the following method. First, an artificial resin tooth that reproduced a Class I cavity (4 mm in diameter, 2 mm in depth) at the central portion of the occlusal surface of the lower right sixth with filled with a dental curable-composition paste to apply the occlusal surface morphology to the filled paste. Thereafter, the artificial resin tooth filled with the dental curable composition was allowed to stand in an incubator at 50°C for 20 minutes to evaluate whether the applied morphology was retained. Samples with a slight change in the applied morphology confirmed were rated as "B", with no change in the applied morphology as "A", and no retention of the applied morphology as "C". Evaluations were performed immediately after the dental curable compositions were prepared and after storage at 37°C for 6 months.

(3) Measurement of Bending Strength of Cured Product

A stainless steel frame was filled with the dental curable composition paste using a filling instrument. In a state of being pressed against the polypropylene, the paste was irradiated three times for 30 seconds each on one surface thereof with a visible ray irradiator Powerlight (manufactured by Tokuyama Corporation) that was closely contacted to the polypropylene such that the entire paste was irradiated with the light. Subsequently, the other side was also irradiated in the same manner three times for 30 seconds each to give a cured product. The cured product was shaped into a rectangular prism of 2×2×25 mm using waterproof abrasive paper #1500. The resulting sample piece was mounted on a tester ("Autograph AG5000D", manufactured by Shimadzu Corporation) and measured for three-point bending fracture strength at a distance of 20 mm between supports and a cross-head speed of 1 mm/min. Then, the bending strength $\sigma_B$ (Pa) was determined according to the following formula, and the average value obtained by evaluating five test pieces was defined as the bending strength. Note that P represents a load (N) at the time of breakage of the test piece, S represents a distance (m) between supports, W represents the width (m) of the test piece, and B represents the thickness (m) of the test piece.

$$\sigma_B = \frac{3PS}{2WB^2}$$

(4) Method of Measuring Hardness of Cured Product

The hardness of the cured product was measured by the method described below. A mold made of polytetrafluoroethylene and having a hole of 6 mmΦ × 1.0 mm was filled with the dental curable composition paste. In a state of being pressed against the polypropylene, the paste was irradiated for 20 seconds on one surface thereof with a visible ray irradiator Powerlight (manufactured by Tokuyama Corporation) to give a cured product. The Vickers hardness of the obtained cured product was determined from the length of a diagonal line of a dent formed in the test piece with a microhardness tester (Model MHT-1, manufactured by MATSUZAWA Co., Ltd.) by pressing a Vickers' indenter thereon with a load of 100 gf and maintaining the load for 30 seconds.

(5) Preparation Method of Dental Curable Composition

<Example 1>

In a polymerizable monomer component (B) composed of GMA (60 parts by mass) and 3G (40 parts by mass), CQ (0.20 parts by mass) as a photosensitizer compound (c1), DMBE (0.35 parts by mass) as a reducing agent (c2), and DPIB (0.3 parts by mass) as a photoacid generator (c3) composed of an aryliodonium salt compound were completely dissolved as polymerization initiators. Thereafter, the obtained solution with an inorganic filler F-1 (200 parts by mass) and an organic-inorganic composite filler CF-1 with a curved surface shape (200 parts by mass) obtained in Production Example 1 was manually mixed in a mortar until uniform, kneaded for 20 minutes, and then degassed to prepare a dental curable composition. The obtained dental curable composition was evaluated for each physical property based on the method described above. The results are shown in Table 4.

<Examples 2-14, Comparative Examples 1-6>

Dental curable compositions of Examples 2-14 and Comparative Examples 1-6 were prepared in the same manner as in Example 1 according to the compositions (parts by mass) shown in Tables 2 and 3. As shown in the footnotes of Tables 2 and 3, the HM ratio indicates the content (mass%) of the polymerizable monomer having a hydrogen-bonding functional group in the polymerizable monomer component (a) or the content (mass%) of the polymerizable monomer having a hydrogen-bonding functional group in the polymerizable monomer (B). In addition, "↑" in Tables 2 and 3 means the same as above. The results of evaluating the physical properties of the obtained dental curable compositions are shown in Table 4.

[Table 2]

| No. | | Organic-inorganic composite filler (A) | | | Polymerizable monomer component (B) | | Polymerization initiator (C) | | | Inorganic filler |
|---|---|---|---|---|---|---|---|---|---|---|
| | | Shortened code/ parts by mass | HM ratio[*1] | Classification | Shortened code/ parts by mass | HM ratio[*2] | Photosensitizer compound (c1) | Reducing agent (c2) | Photoacid generator (c3) | |
| Example | 1 | CF-1/200 | 100 | Porous | GMA/60 3G/40 | 60 | CQ/0.20 | DMBE/0.35 | DPIB/0.3 | F-1/200 |
| | 2 | ↑ | ↑ | ↑ | ↑ | ↑ | ↑ | DMBE/0.35 MDEOA/0.20 | ↑ | ↑ |
| | 3 | CF-2/200 | 50 | ↑ | ↑ | ↑ | ↑ | DMBE/0.35 | ↑ | ↑ |
| | 4 | CF-3/200 | 100 | ↑ | ↑ | ↑ | ↑ | ↑ | ↑ | ↑ |
| | 5 | CF-4/200 | 50 | ↑ | ↑ | ↑ | ↑ | ↑ | ↑ | ↑ |
| | 6 | CF-1/200 | 100 | ↑ | ↑ | ↑ | ↑ | ↑ | DPICH/0.3 | ↑ |
| | 7 | ↑ | ↑ | ↑ | ↑ | ↑ | ↑ | DMBE/0.35 MDEOA/0.20 | ↑ | ↑ |
| | 8 | ↑ | ↑ | ↑ | ↑ | ↑ | ↑ | DMBE/0.35 | DPIP/0.3 | ↑ |
| | 9 | ↑ | ↑ | ↑ | ↑ | ↑ | ↑ | DMBE/0.35 MDEOA/0.20 | ↑ | ↑ |
| | 10 | ↑ | ↑ | ↑ | ↑ | ↑ | ↑ | DMBE/0.35 | DPIFP/0.3 | ↑ |
| | 11 | ↑ | ↑ | ↑ | ↑ | ↑ | ↑ | ↑ | ↑ | ↑ |
| | 12 | CF-2/200 | 50 | ↑ | GMA/30 D-2.6-E/30 3G/40 | 30 | ↑ | ↑ | DPIB/0.3 | ↑ |
| | 13 | CF-3/200 | 100 | ↑ | UDMA/70 3G/30 | 70 | ↑ | ↑ | ↑ | ↑ |
| | 14 | ↑ | ↑ | ↑ | ↑ | ↑ | ↑ | ↑ | DPICH/0.3 | ↑ |

*1 indicates the content (mass%) of the polymerizable monomer having a hydrogen-bonding functional group in the polymerizable monomer component (a) used as a raw material.

*2 indicates the content (mass%) of the polymerizable monomer having a hydrogen-bonding functional group in the polymerizable monomer component (B).

EP 4 166 127 A1

16

[Table 3]

| No. | | Raw material composition | | | | | | | | |
|---|---|---|---|---|---|---|---|---|---|---|
| | | organic-inorganic composite filler (A) | | | Polymerizable monomer component (B) | | Polymerization initiator (C) | | | Inorganic filler |
| | | Shortened code/ parts by mass | HM ratio[*1] | Classif ication | Shortened code/ parts by mass | HM ratio[*2] | Photosensit izer compound (c1) | Reducing agent (c2) | Photoacid generator (c3) | |
| Comparative Example | 1 | CF-1/200 | 100 | ↑ | GMA/60 3G/40 | 60 | ↑ | ↑ | - | ↑ |
| | 2 | ↑ | | | ↑ | | ↑ | DMBE/0.35 MDEOA/0.20 | - | ↑ |
| | 3 | ↑ | | | D-2.6E/60 3G/40 | 0 | ↑ | DMBE/0.35 | - | ↑ |
| | 4 | ↑ | | | GMA/60 3G/40 | 60 | ↑ | ↑ | TCT/0.3 | ↑ |
| | 5 | CF-3/200 | 100 | ↑ | UDMA/70 3G/30 | 70 | ↑ | ↑ | ↑ | ↑ |
| | 6 | CF-6/200 | 50 | Non porous | GMA/6 0 3G/40 | 60 | ↑ | ↑ | - | ↑ |

*1 indicates the content (mass%) of the polymerizable monomer having a hydrogen-bonding functional group in the polymerizable monomer component (a) used as a raw material.

*2 indicates the content (mass%) of the polymerizable monomer having a hydrogen-bonding functional group in the polymerizable monomer component (B).

EP 4 166 127 A1

[Table 4]

| No. | | Evaluation result | | | | | | Bending strength (Mpa) | Vickers hardness |
|---|---|---|---|---|---|---|---|---|---|
| | | Handling property | | | | | | | |
| | | Stickiness | | Dryness | | Shape retention property | | | |
| | | Immediately after preparation | After 6 months | Immediately after preparation | After 6 months | Immediately after preparation | After 6 months | | |
| Example | 1 | A | A | A | A | A | A | 152 | 62 |
| | 2 | A | A | A | A | A | A | 172 | 72 |
| | 3 | A | A | A | A | A | A | 145 | 59 |
| | 4 | A | A | A | A | A | A | 155 | 61 |
| | 5 | A | A | A | A | A | A | 150 | 58 |
| | 6 | A | A | A | A | A | A | 155 | 63 |
| | 7 | A | A | A | A | A | A | 175 | 74 |
| | 8 | A | A | A | A | A | A | 158 | 60 |
| | 9 | A | A | A | A | A | A | 168 | 71 |
| | 10 | A | A | A | A | A | A | 152 | 60 |
| | 11 | A | A | A | A | A | A | 165 | 69 |
| | 12 | A | A | B | A | A | A | 140 | 58 |
| | 13 | A | A | A | A | A | A | 150 | 63 |
| | 14 | A | A | A | A | A | A | 151 | 62 |
| Comparative Example | 1 | C | C | A | A | C | C | 142 | 42 |
| | 2 | C | C | A | A | C | C | 143 | 44 |
| | 3 | A | A | C | C | B | B | 120 | 41 |
| | 4 | C | C | A | A | C | C | 148 | 60 |
| | 5 | C | C | A | A | C | C | 150 | 62 |
| | 6 | A | A | B | B | A | A | 90 | 41 |

As shown in Table 4, the dental curable compositions in Examples 1-14 that satisfy the conditions defined in the present invention had little stickiness and dryness and exhibited good shape retention properties both immediately after preparation and after long-term storage, and the cured products thereof exhibited high bending strength and Vickers hardness. In particular, the dental curable compositions in Examples 2, 7, 9, and 12 in which the aromatic tertiary amine and the aliphatic tertiary amine were used in combination as the reducing agent (c2) exhibited high values of the bending strength and the Vickers hardness of the cured products.

On the other hand, the dental curable compositions in Comparative Examples 1 and 2, in which aryliodonium salts were not contained as the photoacid generator (c3) in the polymerization initiator, had large stickiness of the paste, a low ability to retain the applied morphology, and a lower Vickers hardness of the cured products than when the aryliodonium salts were contained. In addition, the dental curable composition of Comparative Example 3, which does not contain a hydrogen-bonding functional group as a polymerizable monomer, had a large dryness of the pastes (as a problem before the paste became sticky), making them difficult to use, and the bending strength of the cured products was also decreased. Further, the dental curable compositions in Comparative Examples 4 and 5, in which a compound other than the aryliodonium salt was contained as the photoacid generator, had high bending strength and Vickers hardness of the cured product but did not exhibit improved handling property. Furthermore, the dental curable composition in Comparative Example 6, which contains a nonporous, irregular-shaped organic-inorganic composite filler, exhibited a relatively good handling property without aryliodonium salt, but the cured product had significantly lower bending strength and lower Vickers hardness.

**Claims**

1. A dental curable composition comprising:

   a porous organic-inorganic composite filler (A)

   which comprises:

   an organic resin component obtained by polymerizing a polymerizable monomer component (a) including at least 50 mass% of a polymerizable monomer having a hydrogen-bonding functional group, and
   an inorganic component composed of a plurality of
   inorganic particles, and

   in which a total pore volume of pores having a pore diameter in a range of 1-500 nm, as measured by a nitrogen adsorption method, is 0.01-0.30 cm$^3$/g;

   a polymerizable monomer component (B) including at least 10 mass% of a polymerizable monomer having a hydrogen-bonding functional group; and
   a polymerization initiator (C),
   wherein the polymerization initiator (C) comprises, as photopolymerization initiators, a photosensitizer compound (c1), a reducing agent (c2), and a photoacid generator (c3) composed of an aryliodonium salt compound; and
   a content of the aryliodonium salt compound is 0.1-5 parts by mass with respect to 100 parts by mass of a total mass of the polymerizable monomer component (B).

2. The dental curable composition according to claim 1,
   wherein the organic-inorganic composite filler (A) comprises a resin layer composed of a cured product of the polymerizable monomer component (a) as the organic resin component, and a plurality of inorganic primary particles having an average particle diameter of 10-1000 nm as the inorganic component, and comprises a porous organic-inorganic composite aggregated particle having an aggregate structure in which the plurality of inorganic primary particles are bonded to each other via the resin layer so as to form voids.

3. The dental curable composition according to claim 1 or 2, wherein the reducing agent (c2) comprises an aromatic tertiary amine compound and an aliphatic tertiary amine compound.

4. The dental curable composition according to any one of claims 1 to 3, further comprising an inorganic filler (D) composed of a plurality of inorganic particles having an average particle diameter of 10-1000 nm.

5. The dental curable composition according to claim 4, wherein contents of the organic-inorganic composite filler (A) and the inorganic filler (D) are each 100-300 parts by mass with respect to 100 parts by mass of a total mass of the polymerizable monomer (B), and a total content of the organic-inorganic composite filler (A) and the inorganic filler (D) is 250-550 parts by mass with respect to 100 parts by mass of the total mass of the polymerizable monomer (B).

6. The dental curable composition according to claim 4 or 5, wherein the inorganic particles contained in the organic-inorganic composite filler (A) and the inorganic particles constituting the inorganic filler (D) are both spherical or substantially spherical in shape.

# EP 4 166 127 A1

<table>
<tr><td colspan="2">INTERNATIONAL SEARCH REPORT</td><td>International application No.</td></tr>
<tr><td colspan="2"></td><td>PCT/JP2021/016906</td></tr>
</table>

A.  CLASSIFICATION OF SUBJECT MATTER
Int.Cl. A61K6/818(2020.01)i, A61K6/831(2020.01)i, A61K6/887(2020.01)i
FI: A61K6/887, A61K6/818, A61K6/831

According to International Patent Classification (IPC) or to both national classification and IPC

B.  FIELDS SEARCHED

Minimum documentation searched (classification system followed by classification symbols)
Int.Cl. A61K6/818, A61K6/831, A61K6/887

Documentation searched other than minimum documentation to the extent that such documents are included in the fields searched
| | |
|---|---|
| Published examined utility model applications of Japan | 1922–1996 |
| Published unexamined utility model applications of Japan | 1971–2021 |
| Registered utility model specifications of Japan | 1996–2021 |
| Published registered utility model applications of Japan | 1994–2021 |

Electronic data base consulted during the international search (name of data base and, where practicable, search terms used)
JSTPlus/JMEDPlus/JST7580(JDreamIII)

C.  DOCUMENTS CONSIDERED TO BE RELEVANT

| Category* | Citation of document, with indication, where appropriate, of the relevant passages | Relevant to claim No. |
|---|---|---|
| Y | JP 2015-105254 A (TOKUYAMA DENTAL CORPORATION) 08 June 2015 (2015-06-08), claims, examples, fig. 1, paragraphs [0002], [0099] | 1-6 |
| Y | JP 2019-183111 A (TOKUYAMA DENTAL CORPORATION) 24 October 2019 (2019-10-24), claims, examples 5-11, 17-23, paragraphs [0006], [0137], [0156], [0157] | 1-6 |
| Y | JP 2014-177443 A (TOKUYAMA DENTAL CORPORATION) 25 September 2014 (2014-09-25), claims 13, 14, paragraphs [0181]-[0191], table 3, paragraph [0153] | 1-6 |
| Y | JP 2005-213231 A (TOKUYAMA CORPORATION) 11 August 2005 (2005-08-11), claims 1, 2, paragraphs [0011], [0016], [0060] | 1-6 |

☒ Further documents are listed in the continuation of Box C.　　☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 07 June 2021 | 22 June 2021 |

| Name and mailing address of the ISA/ | Authorized officer |
|---|---|
| Japan Patent Office 3-4-3, Kasumigaseki, Chiyoda-ku, Tokyo 100-8915, Japan | Telephone No. |

Form PCT/ISA/210 (second sheet) (January 2015)

| International application No. |
| --- |
| PCT/JP2021/016906 |

C (Continuation).    DOCUMENTS CONSIDERED TO BE RELEVANT

| Category* | Citation of document, with indication, where appropriate, of the relevant passages | Relevant to claim No. |
| --- | --- | --- |
| Y | JP 2010-208964 A (TOKUYAMA DENTAL CORPORATION) 24 September 2010 (2010-09-24), claim 1, paragraph [0082] | 1-6 |
| Y | WO 2010/090011 A1 (TOKUYAMA DENTAL CORPORATION) 12 August 2010 (2010-08-12), claim 1, paragraphs [0057], [0129] | 1-6 |
| Y | WO 2017/154983 A1 (TOKUYAMA DENTAL CORPORATION) 14 September 2017 (2017-09-14), claims 1-5, paragraph [0076] | 1-6 |

Form PCT/ISA/210 (second sheet) (January 2015)

**INTERNATIONAL SEARCH REPORT**
Information on patent family members

International application No.

PCT/JP2021/016906

```
JP 2015-105254 A    08 June 2015         (Family: none)

JP 2019-183111 A    24 October 2019      (Family: none)

JP 2014-177443 A    25 September 2014    (Family: none)

JP 2005-213231 A    11 August 2005       (Family: none)

JP 2010-208964 A    24 September 2010    (Family: none)

WO 2010/090011 A1   12 August 2010       US 2011/0288195 A1
                                         claim 1, paragraphs [0074], [0172]
                                         EP 2394628 A1
                                         CN 102300545 A

WO 2017/154983 A1   14 September 2017    EP 3427716 A1
                                         claims 1-5, paragraph [0104]
                                         CN 108697586 A
                                         KR 10-2018-0122614 A
```

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000080013 A
- WO 2015125470 A
- WO 2011115007 A
- JP 6219146 B